# EUROPEAN PATENT SPECIFICATION

(11) **EP 0 874 635 B1**
(45) Date of publication and mention of the grant of the patent: **19.03.2003**
(21) Application number: 96943630.2
(22) Date of filing: 04.12.1996
(51) Int. Cl.: A61K 35/14, A61K 35/18

(54) **Nonimmunogenic platelet and red blood cell compositions**
Nicht-immunogene Blutplättchen und rote Blutzellen enthaltende Zubereitungen
Compositions nonimmunogènes contentant des plaquettes et des globules rouges

(30) Priority: 04.12.1995 US 566847
(43) Date of publication of application: 04.11.1998
(73) Proprietor: Puget Sound Blood Center, Seattle, Washington 98104-1256 (US)
(72) Inventor: SLICHTER, Sherrill, J., Dockton, WA 98070 (US)
(74) Representative: Gowshall, Jonathan Vallance
(86) International application number: US9619467
(87) International publication number: WO97020570

(56) References cited:
- EP-A- 0 233 112
- EP-A- 0 530 688
- WO-A-87/00053
- WO-A-91/04088
- PATENT ABSTRACTS OF JAPAN vol. 006, no. 078 (C-102), 15 May 1982 & JP 57 014524 A (ASAHI CHEM IND CO LTD), 25 January 1982,
- PATENT ABSTRACTS OF JAPAN vol. 006, no. 139 (C-116), 28 July 1982 & JP 57 062222 A (ASAHI CHEM IND CO LTD), 15 April 1982,

## Description

### Technical Field

The present invention relates generally to blood products, and more specifically to non-immunogenic and toleragenic platelet and red blood cell compositions, and methods of producing the same.

### Background of the Invention

Patients with a variety of disorders receive intermittent or chronic transfusion support or require tissue grafting to replace a defective organ. Individuals requiring transfusion support may have either a genetic or acquired deficiency of one or more blood components that require replacement therapy. Many different products prepared from blood are available for transfusion, including both cellular and plasma components. However, repeated exposure to blood products often results in recipient recognition of the foreign transfused antigens. For instance, alloimmune platelet refractoriness occurs in up to 30%-60% of patients requiring repeated platelet transfusions. Such immune recognition of the foreign antigens results in a failure to achieve a benefit from the transfusion and in some circumstances may even cause a transfusion reaction with adverse consequences to the recipient.

Several approaches have been used to either prevent or delay alloimmunization. The majority of the techniques involve giving immunosuppressive therapy to the transfusion recipient to prevent recognition of the transfused foreign antigens. Such immunosuppressive therapy is often inadequate to suppress the recognition process resulting in alloimmunization in spite of the treatment. Furthermore, the immunosuppressive therapy may have undesirable side effects including organ toxicity and immunosuppression of desirable responses such as recognition and destruction of pathogenic bacteria.

Once an immune response to foreign antigens has occurred, there is little evidence that any immunosuppressive therapy is beneficial. Continued adequate transfusion support is possible only if antigen matching between donor and recipient is achieved. Often a matched donor is not available or for some transfusion products so little is known about the antigen systems involved in the immune response that laboratory methods are not available to appropriately select a matched donor.

An alternative approach to preventing alloimmunization, other than immunosuppressing the recipient, is to reduce the immunogenicity of the transfused product. As all transfused blood products are immunogenic and will eventually induce an immune response in most transfused recipients, any procedure that can prevent or at least delay immunization is beneficial. Selecting only antigen compatible donors beginning with the first transfusion is possible in some circumstances, but for the majority of patients not enough donors are available to continue this process or a matching procedure does not exist.

For organ grafting, because there is persistent exposure to foreign tissue antigens, eventual rejection of the grafted tissue occurs. To prevent graft rejection several approaches have been used: recipient immunosuppression, matching tissue antigens of donor and recipient, reducing the immunogenicity of the grafted tissue, or inducing a state of tolerance in the recipient to the foreign antigens of the graft. Furthermore, depending on the tissue being grafted different approaches may be required to achieve a successful graft and combined therapies may be additive in their beneficial effects. For example, in bone marrow transplantation massive doses of chemo-radiotherapy are given to the recipient to destroy the recipient's autologous marrow and to induce immunosuppression to allow engraftment of the donor marrow. Even better results are obtained if marrow donor and recipient are related and well-matched for the major histocompatibility antigen system (HLA). Although post-marrow grafting immunosuppression is usually given, it is for only a limited time.

In contrast, for kidney grafting lesser degrees of immunosuppressive therapy are required to avoid unacceptable marrow and gastrointestinal toxicity. Furthermore, continuous post-grafting immunosuppression is required. Often a related kidney donor is not available, and lesser degrees of HLA matching between donor and recipient are more often accepted than for bone marrow transplantation.

Another major difference between these two types of tissue grafting is the effect of prior transfusions on engraftment. For kidney graft recipients, prior transfusions, particularly from the intended kidney donor, are beneficial apparently by inducing some degree of tolerance to the subsequent kidney graft. However, prior blood transfusions before marrow grafting, especially if the blood has come from the intended marrow donor, markedly increase the risk of graft rejection. Thus, although there are similarities in procedures to enhance organ grafts (immunosuppression and donor-recipient HLA matching) there are clear differences in (1) the amounts, type, and duration of immunosuppression required; (2) the acceptance of non-HLA identity between organ donor and recipient; and, (3) the effects of prior transfusions on enhancing or impairing a subsequent organ graft. Furthermore, even the best combined therapies are not always successful in ensuring a successful organ graft, and there may be substantial toxicities associated with the therapies being used.

Besides using HLA matching and recipient immunosuppression, efforts to directly reduce immunogenicity of the engrafted tissue or to induce tolerance in the recipient, other than by prior blood transfusions in kidney recipients, have been limited. In bone marrow transplantation efforts to purify or enrich the marrow graft for stem cells and eliminate T-lymphocytes that may be responsible for graft vs. host disease (a post-grafting complication) have often resulted in a transplanted marrow that has failed to engraft. Some investigators have stored or cultured the graft *in vitro* prior to transplantation (skin grafting) to facilitate engraftment. Most of these latter methods to enhance organ grafting have had limited success.

It would be advantageous to avoid immune recognition by the recipient of incompatible donor antigens and the consequent destruction of allogeneic tissue following transfusion or transplantation. The present invention fulfills this need and further provides other related advantages.

### Summary of the Invention

The present invention provides non-immunogenic and/or toleragenic platelet and/or red blood cell compositions. Such compositions are substantially free of white blood cells.

In a related aspect of the present invention, methods of preparing such non-immunogenic and toleragenic platelet compositions are provided, comprising centrifuging a platelet-rich blood product one or more times and recovering the supernatant; and filtering the supernatant and recovering the filtrate to produce the platelet composition. The step of centrifuging may comprise one or two soft spins, with or without a hard spin performed at any point in the platelet preparation process. The step of filtration may be performed after the step of centrifugation or between the first and second soft spins. Alternatively, the step of filtration may be performed prior to the step of centrifugation.

Within another related aspect, a method of preparing a non-immunogenic and toleragenic platelet composition comprises: (a) performing a first soft spin on a platelet-rich blood product and recovering a supernatant platelet composition; (b) performing a second soft spin on the platelet composition and recovering a supernatant platelet composition; (c) performing a hard spin on the platelet composition and recovering a supernatant platelet composition; and (d) filtering the platelet composition and recovering a filtrate platelet composition; wherein step (a) is performed prior to steps (b), (c) and (d) and wherein steps (b), (c) and (d) may be performed in any order.

In yet another related aspect, a method of preparing a non-immunogenic and toleragenic platelet composition comprises: (a) performing a hard spin on a platelet-rich blood product and recovering a supernatant platelet composition; (b) performing a soft spin on the platelet composition and recovering a supernatant platelet composition; and (c) filtering the platelet composition and recovering a filtrate platelet composition; wherein step (a) is performed prior to steps (b) and (c) and wherein steps (b) and (c) may be performed in either order.

In another aspect of the present invention, a non-immunogenic platelet composition is provided. The composition may be produced by either (a) centrifuging a platelet-rich blood product, recovering the supernatant and then exposing the composition to UV irradiation; or by (b) filtering the platelet-rich blood product, recovering the filtrate and then exposing the filtrate to UV irradiation. Any of UV-A, UV-B or UV-C irradiation may be used with or without a photoadditive agent (*e.g.,* psoralin), although UV-B is preferred. It will be evident that the steps of centrifugation or filtration and UV irradiation may be performed in either order.

In any of the methods described above, suitable platelet-rich blood products include whole blood, platelet-rich plasma, platelet concentrates, apheresis platelets and bully-coat prepared platelets.

In another aspect, the present invention provides a method of preparing a non-immunogenic and/or toleragenic red blood cell composition, comprising: (a) centrifuging a red blood cell-rich blood product and recovering the lower layer of red cells; and (b) subsequently filtering the lower layer and recovering the filtrate to produce a non-immunogenic and toleragenic red blood cell composition. Alternatively, the filtration can be performed first, followed by the centrifugation step.

In further aspects of the present invention, treatments are provided for inducing immunologic tolerance in a patient to transfused or transplanted tissue. In one such aspect, the treatments comprise administering to a patient a non-immunogenic and toleragenic platelet composition, said composition being substantially free of white blood cells, in an amount sufficient to induce immunologic tolerance to subsequently transfused or transplanted tissue. In another such aspect, the treatments comprise administering to a patient a non-immunogenic and toleragenic red blood cell composition, said composition being substantially free of white blood cells, in an amount sufficient to induce immunologic tolerance to subsequently transfused or transplanted tissue. In various embodiments, the transfused or transplanted tissue may be derived from bone marrow, blood, skin, pancreas, bone, liver, heart, lung, kidney or cornea. Within preferred embodiments, the transfused or transplanted tissue is blood.

### Detailed Description of the Invention

Prior to setting forth the invention, it may be helpful to the understanding thereof to set forth definitions of certain terms to be used hereinafter.

"Non-immunogenic" as used herein refers to a characteristic of compositions which, when administered to a human, are not associated with either the development of a humoral (antibody) or cellular immune response.

"Toleragenic" as used herein refers to the capacity of a composition to generate an immunologic response consisting of specific non-reactivity of the immune system to a given antigen that, in other circumstances, can induce cell-mediated or humoral immunity.

"Substantially free" as used herein refers to a composition that contains at least a two-three log reduction in the number of cells of a given type (*e.g.*, white blood cells) from baseline, preferably to a residual level of 10⁵ per transfusion (as determined by manual counting using a Neubauer chamber with propidium-iodide staining).

As noted above, the present invention provides non-immunogenic and/or toleragenic platelet compositions, the compositions being substantially free of white blood cells. Critical to achieving such compositions is the use of a combination of filtration and centrifugation steps, which may be performed in either order. In a preferred embodiment, one or more centrifugation steps are performed prior to a filtration step. In addition, as noted above, the present invention provides non-immunogenic platelet compositions. Such compositions are produced through either a combination of centrifugation and UV irradiation or filtration and UV irradiation, which steps can be performed in either order. The present invention also provides non-immunogenic and/or toleragenic red blood cell compositions, which may be produced through a combination of centrifugation and filtration steps (which may be performed in either order).

Within the context of the present invention, platelet compositions may be generated from any platelet-rich product such as whole blood, platelet-rich plasma platelet concentrates, apheresis platelets or buffy-coat prepared platelet concentrates. Similarly, red blood cell compositions may be prepared from any red blood cell-rich product, such as whole blood, packed red blood cells or aphoresis red cells. It will be apparent to those of ordinary skill in the art that it may be advantageous to perform one or more steps prior to centrifugation and/or filtration, depending on the starting material employed. For example, whole blood may be diluted (*e.g.*, 1:1) with Ringer's Citrate Dextrose (RCD) prior to centrifugation and filtration to enhance separation of the platelets from red blood cells. RCD may be prepared by adding 20ml of 0.2micron filtered 1M sodium citrate in sterile, non-pyrogenic, injectable water to a 500ml bag of 5% Dextrose in Ringer's Injection solution.

It has been found within the context of the present invention that, except when starting with buffy coat prepared platelet concentrates, both centrifugation and filtration are required to generate a non-immunogenic and toleragenic product. For buffy coat platelets, it has been found that filtration as described herein is sufficient to generate a non-immunogenic and toleragenic product. Without wishing to be bound by any particular theory, it is believed that white blood cell contamination is a significant source of immunogenicity in blood-derived products. Within the present invention, it has been found that blood contains two distinct populations of white blood cells, and that a combination of centrifugation and filtration steps is necessary to render the product substantially free of white blood cells.

For generating a non-immunogenic and toleragenic product, at least one centrifugation step is preferably performed prior to filtration. A first step of centrifugation as described herein is performed to separate red blood cells from platelets, and to remove some of the white blood cells. Such separation may generally be achieved using one or more soft spins and/or a hard spin. As used herein, the term "soft spin" refers to centrifugation performed at 180 to 2000 xg, depending on the volume of blood being processed. Preferably, centrifugation is at about 250 xg for a blood volume of 30 to 60 ml. Centrifugation times of 4 to 30 minutes are sufficient, depending on the blood volume being processed and the g force used. For a blood volume of 30 to 60 ml, 10 minutes at 250 xg is preferred. The term "hard spin" refers to centrifugation at 500 to 6000 xg for 4 to 40 minutes, depending upon the volume being processed, preferably about 900 xg for about 10 minutes for a volume of 30 to 60 ml. Any of a variety of commercially available centrifuges, such as a swinging bucket rotor centrifuge (IEC, Needham Heights, MA), may be employed for the centrifugation step(s).

The initial step of centrifugation may be a soft spin or a hard spin. In one preferred embodiment, an initial soft spin is followed by a second soft spin, a hard spin and a filtration step, which may be performed in any order. In another preferred embodiment, an initial hard spin is followed by a soft spin and a filtration step, which may be performed in either order. The order of steps in these preferred embodiments is provided in Table I, below.

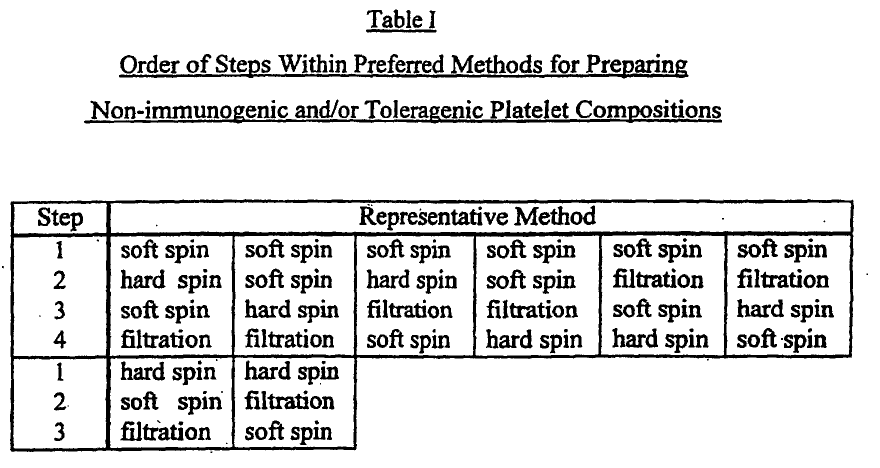

While these steps are generally sufficient to generate a non-immunogenic and toleragenic platelet composition, it will be apparent that other centrifugation and/or filtration steps may also be performed at any point within a series of steps provided above.

For example, to prepare a non-immunogenic and toleragenic platelet composition, an initial soft spin may be performed as described above to separate red blood cells from platelets, and to remove some of the white blood cells. A soft spin performed on whole blood results in a lower layer containing red blood cells, an upper layer containing primarily platelets and plasma, and an interface containing white and red blood cells. For preparing a non-immunogenic and toleragenic red blood cell composition, the upper layer is removed, and it may be filtered directly, as described below.

A hard spin may then be performed to concentrate the platelets. Preferably, the hard spin is performed after the first soft spin and filtration, resulting in the generation of a lower layer of platelet concentrate, and allowing the removal of plasma. The platelet concentrate may then be resuspended and subjected to a second soft spin after filtration for more complete removal of white blood cells. However, the second soft spin to remove additional white blood cells may also be performed on the platelet-rich plasma prior to filtration, after filtration prior to the hard spin, or as described above after both the filtration and hard spin have been performed.

Alternatively, a platelet preparation (a buffy coat platelet concentrate) may be prepared by first performing a hard spin on the whole blood. A hard spin separates the whole blood into an upper layer containing mainly plasma, a lower layer containing red blood cells, and an interface containing white cells, red cells, and platelets. The plasma is removed and the interface containing white cells, platelets and red cells is removed and subjected to a soft centrifugation. Either individual units are subjected to the soft spin, or the soft spin is performed after pooling of individual buffy coats prepared from other blood donations. This soft centrifugation removes white and red blood cells. These buffy coat platelets are then subjected to filtration.

To prepare non-immunogenic and toleragenic red blood cells, at least one centrifugation step (either a hard spin or a soft spin) and a filtration step may be performed. In a preferred embodiment, the lower layer of red cells obtained either following a soft or hard centrifugation of the whole blood is removed and filtered. Alternatively, a red blood cell-rich product such as whole blood can be filtered and then subjected to a hard or soft centrifugation with removal of the lower layer of red blood cells. Filtration may be performed using any of a variety of apparatus and procedures known to those of ordinary skill in the art. For example; suitable filters include Pall filters BPF-4 and RC-10 (Pall Biomedical Products, East Hills, NY).

Filtration step(s) as described herein are performed to further reduce the number of white blood cells. Filtration may be performed using any of a variety of apparatus and procedures known to those of ordinary skill in the art. For example, suitable filters include Pall filters (Pall Biomedical Products, East Hills, NY), such as PLF-1, PLF-10, PL-50 and PL-100, platelet filter TF*IG500 (Terumo, Somerset, NJ) and platelet filter 4C2477 (Fenwal/Baxter, Chicago, IL), with PLF-10 being preferred.

To prepare a platelet composition, if one or more centrifugation steps are performed prior to filtration, the collected supernatant is passed through the filtration apparatus. Within the various embodiments, the step of filtration may be performed a second time, with an expectation of a slightly greater degree of leuko-reduction. However, to achieve the advantages described herein, it is not necessary to filter the product more than once. As noted above, filtration may be performed prior to centrifugation. In such embodiments, the filtrate is then subjected to centrifugation as described above.

The centrifugation and filtration steps described above are sufficient to generate a non-immunogenic and toleragenic product. A centrifuged and/or filtered platelet composition may, however, be further treated by exposure to UV irradiation. Although any of UV-A, UV-B or UV-C irradiation may be used, UV-B is preferred. UV-A represents a wavelength of 320-400 nm, UV-B represents a wavelength of 290-320 nm, and UV-C represents a wavelength of 200-290 nm. The total dose of UV-B irradiation generally results in an exposure of between approximately 600 and 1632 mJ/cm², with approximately 612 mJ/cm² being preferred. The total dose of UV-C irradiation is preferably between 12 and 36 mJ/cm². The intensity of the irradiation (and therefore a determination of the total exposure) may be obtained through use of a radiation detector (International Light, Inc. Newburyport, MA).

The UV-A, UV-B or UV-C irradiation (with or without photoadditive agents (*e.g.*, psoralin)) may be performed at any time in the preparation of the platelet and/or red blood cell composition. It will be evident that a wide variety of apparatus may be used to provide the UV exposure described herein. Such apparatus include a germicidal lamp (General Electric) and a Haemonetics (Braintree, MA) UV-B irradiation device. Upon preparation of a UV-irradiated platelet or red blood cell composition, one can confirm its non-immunogenic and toleragenic properties *in vitro* by performing mixed lymphocyte culture (MLC) experiments and determining that the residual lymphocytes are no longer able to stimulate or respond in MLC.

As noted above, the non-immunogenic and toleragenic platelet and red blood cell compositions described herein may be used for tolerance induction. A administration of the compositions for this purpose will be evident to those skilled in the art. Tolerance is induced by administering transfusions, generally repeated transfusions, of a platelet or red cell composition to a recipient. Transfusions are usually given on a weekly basis, but other schedules are suitable. Generally, from one to eight transfusions are provided, with at least three transfusions being preferred.

To determine whether alloimmune platelet refractoriness has occurred, both platelet responses and antibody measurements are useful. Platelet responses are measured by determining pre- and post-transfusion platelet counts and calculating platelet increments, % platelet recovery, or corrected count increments. Antibody measurements are made using a variety of tests including platelet and/or lymphocytotoxic antibody tests. Such tests are well known to those skilled in the art.

Alternatively, to determine whether only alloimmunization has occurred, antibody measurements are used. Antibody measurements are made using a variety of tests including platelet and/or lymphocytotoxic antibody tests.

The following examples are offered by way of illustration and not by way of limitation.

### EXAMPLES

### Example 1

### Preparation of Platelet Compositions

This Example illustrates the preparation of non-immunogenic and/or toleragenic platelet compositions.

Twenty-seven milliliters of dog blood is drawn via a jugular vein puncture with an 18G 11/2" needle (Becton-Dickinson Immunocytometry Systems, San Jose, CA) into a 30ml syringe (Becton-Dickinson) containing 3ml of Acid Citrate Dextrose formula A (ACD-A anti-coagulant solution). The blood is then mixed with the ACD-A (Fenwal/Baxter, Chicago, IL) by inverting the syringe three times. The anti-coagulated blood is expressed into a 150ml transfer pack (Baxter) to which 30ml of Ringer's Citrate Dextrose (RCD) has been added. RCD is prepared by adding 20ml of 0.2micron filtered (Gelman Sciences, Ann Arbor, MI) 1M sodium citrate (Mallinckrodt, Inc., Paris, KY) in sterile, non-pyrogenic, injectable water (Baxter) to a 500ml bag of 5% Dextrose in Ringer's Injection solution (Baxter). The 150ml pack is inverted three times to mix the ACD blood and RCD and placed in a swinging bucket rotor centrifuge (IEC) for 10 minutes at 250 xg. Platelet rich plasma (PRP) is expressed along with the buffy coat down to the red cell layer via a plasma press (Fenwal) into a second 150ml transfer pack. The PRP is filtered via gravity flow through a PLF-1 filter (Pall) connected to a third 150ml transfer pack. The third transfer pack containing the leucopoor PRP cells is centrifuged in a swinging bucket rotor centrifuge at 900 xg for 10 minutes. Plasma/RCD is expressed into a fourth 150ml transfer pack via a plasma press and saved. The remaining cell pellet is gently massaged in the third transfer pack to resuspend it in the residual solution left by the plasma press. 300µCi of sterile, liquid sodium ⁵¹chromate (Amersham) is added and the bag is gently inverted five times to mix the solution. The bag is left to incubate at room temperature for 60 minutes. The unbound 51CR is washed away by transferring the set aside plasma/RCD in the 150ml transfer pack into the 150ml transfer pack containing the 51CR labeled platelets. The components are mixed by inverting three times, and the radioactive platelets pelleted by spinning for 10 minutes at 900 xg in a swinging bucket rotor centrifuge. The radioactive plasma is decanted and 6ml of RCD is added to the 150ml pack. The pelleted platelets are resuspend by gentle massage, decanted into a 13ml sterile snap cap tube (Falcon #1054) and centrifuged in a swinging bucket for five minutes at 180 xg. The supernatant is aspirated to within 5 mm of the RBC/WBC pellet into a 10ml syringe using a 10cm long piece of transfer pack tubing attached to the end. Five milliliters is used for injection.

This method of preparation was used to prepare the control platelets *(i.e.,* without filtration or UV irradiation, but leuko-reduced by centrifugation) (row 1, Table III), the leuko-reduced composition prepared by centrifugation and UV irradiation (the filtration step described above was not performed, but rather UV irradiation was performed as described in Example 2) (row 4, Table III), and the composition leuko-reduced by both filtration and centrifugation (row 5, Table III).

### Example 2

### Preparation of Platelet Compositions

This Example illustrates the preparation of further non-immunogenic and/or toleragenic platelet compositions.

Twenty-seven milliliters of dog blood is drawn via a jugular vein puncture with an 18G 11/2" needle (Becton-Dickinson) into a 30ml syringe (Becton-Dickinson) containing 3ml of Acid Citrate Dextrose formula A (ACD-A anti-coagulant solution). The blood is then mixed with the ACD-A (Fenwal/Baxter) by inverting the syringe three times. The anti-coagulated blood is expressed into a 150ml transfer pack (Baxter) to which 30ml of Ringer's Citrate Dextrose (RCD) has been added. RCD is prepared by adding 20ml of 0.2micron filtered (Gelman) 1M sodium citrate (Mallinckrodt) in sterile, non-pyrogenic, injectable water (Baxter) to a 500ml bag of 5% Dextrose in Ringer's Injection solution (Baxter). The 150ml pack is inverted three times to mix the ACD blood and RCD and placed in a swinging bucket rotor centrifuge (IEC) for 10 minutes at 250 xg. Platelet rich plasma (PRP) is expressed along with the buffy coat down to the red cell layer via a plasma press (Fenwal) into a second 150ml transfer pack. The PRP is filtered via gravity flow through a PLF-1 filter (Pall) connected to another 150ml transfer pack. If the product is to be UV-irradiated, the leucopoor PRP is transferred to a 300ml uv transmissible bag (Stericell by Terumo, Somerset, NJ) and illuminated with a monitored dose (International Light Inc., Newburyport, MA) of 612mJ/cm² uvB with agitation on a Haemonetics Platelet Treatment System (Braintree, MA). The UV irradiated leucopoor PRP is transferred to a 150ml pack in which the cells are pelleted in a swinging bucket rotor centrifuge at 900 xg for 10 minutes. Plasma/RCD is expressed into a 150ml transfer pack via a plasma press and saved for later. The remaining cell pellet is gently massaged in the transfer pack to resuspend it in the residual solution left by the plasma press. The platelet suspension is decanted into a 13ml sterile snap cap centrifuge tube (Falcon #2054) and two sequential 3ml rinses of the pack with RCD in a 3ml syringe (Becton-Dickinson) are added to the snap cap tube. This tube is spun in a swinging bucket (IEC) at 180xg for five minutes.

The PRP is removed with a transfer pipette (Globe Scientific, Inc., Paramus, NJ) to within 5mm of the RBC/WBC pellet into another snap cap tube, while the tube containing the RBC/WBC pellet is saved. 300uCi of sterile, liquid sodium 51chromate (Amersham) is added and gently vortexed for one second. The tube is left to incubate at room temperature for 60 minutes. The unbound 51CR is washed away by transferring the 1CR/PRP back to the 150ml pack containing plasma/RCD. The components are mixed by inverting three times, and the radioactive platelets pelleted by spinning for 10 minutes at 900 xg. in a swinging bucket rotor centrifuge. The radioactive plasma is decanted and 6ml of RCD added to the 150ml pack. The pelleted platelets are resuspended by gentle massage, decanted into a 13ml sterile snap cap tube and centrifuged in a swinging bucket for five minutes at 180 xg. Using a transfer pipette, the platelets are transferred into the saved 13ml snap cap tube containing the pelleted RBC/WBC. The platelets and RBC/WBC are mixed gently by squeezing and releasing the bulb of the transfer pipette 5 to 10 times and aspirated into a 10ml syringe using a 10cm long piece of transfer pack tubing attached to the end. Five milliliters is used for injection.

This method of preparation was used to prepare the leuko-reduced composition prepared by filtration (row 2, Table III) and the composition prepared by filtration and UV irradiation (row 3, Table III).

### Example 3

### Prevention of Platelet Alloimmunization

This Example illustrates the ability of representative non-immunogenic and/or toleragenic platelet compositions to prevent platelet alloimmunization.

Pairs of dogs were selected as donor and recipient. Each recipient's response to transfused radiolabeled donor platelets was determined according to a specified transfusion program. In order to detect immunization to the primary donor, up to 8 weeks of treated platelet transfusions were given form the primary donor (1°). In order to evaluate tolerance induction to the primary donor, up to 8 additional weeks of control platelet transfusions from the primary donor were given, if the dog had not become refractory to their 8 weeks of treated platelet transfusions. In order to evaluate tolerance induction to other donors, up to 8 weeks of control platelet transfusions were given from a secondary (2°) and a tertiary donor (3°). For each of these transfusion schedules, 51 Chromium radiolabeled donor platelets were given every week for up to 8 weeks or until the recipient became refractory, defined as less than 5% recovery of donor platelets in the transfused recipient at 24 hours post-injection on 2 sequential occasions. At time of refractoriness, transfusions from the next donor were initiated. In addition to determining platelet refractoriness by radiolabeled platelet survival measurements, some of the transfused recipients also had measurements performed for recipient antibodies against donor platelets, lymphocytes, and red cells by flow cytometry techniques using a FACScan (Becton Dickinson Immunocytometry Systems, San Jose, CA).

Table II depicts the results of the cell counts done on the platelet products (per dose) prior to injection as prepared by the techniques outlined under Examples 1 and 2.

**Table II**

| Range of Total Cell Counts of Transfusion Products | | | | | | |
|---|---|---|---|---|---|---|
| | Control Platelets | Treated Platelets | | | | |
| | LR by CENT | LR by Fx1 | LR by Fx2 | LR by Fx1 & UVI | LR by Cent & UVI | LR by Fx1 *plus* LR & ER by CENT |
| PLAT* | 1.2 - 3.4 x 10⁹ | 1.8 - 4.7 x 10⁹ | 1.0 - 3.0 x 10⁹ | 1.0 - 2.2 x 10⁹ | 1.3 - 2.8 x 10⁹ | 0.8 - 4.0 x 10⁹ |
| | | | | | | |
| WBC** | <3 x 10³*** to 1 x 10⁴ | <3 x 10³ to to 1 x 10⁵ | <3 x 10³ | <3 x 10³ to 1 x 10⁵ | <3 x 10³ to 1 x 10⁴ | <3 x 10³ |

| | | | | | | |
|---|---|---|---|---|---|---|
| * Determined by automated cell counter (Coulter). | | | | | | |
| ** Determined by manual counting (Neubauer chamber with Propidium-Iodide staining). | | | | | | |
| *** Limit of sensitivity by the method used for WBC counting | | | | | | |

Table III depicts the results of the transfusion experiments. Using modified platelet transfusions from the primary donor, neither the control platelet products that were white cell reduced by centrifugation nor the filtered products-whether filtration was performed either once or twice- were able to prevent platelet alloimmunization, compared to the products that received more than one type of modification; *i.e.,* platelets that were white cell reduced by centrifugation and then either UV irradiated or filtered, or leukocyte-reduced by filtration and then UV irradiation. Within the group of doubly-modified products, none of these products were significantly different from each other in their ability to prevent primary alloimmunization, and they were equally efficacious in maintaining tolerance to control platelet products from their primary donors. However, only the platelet products that were white cell reduced by centrifugation and also further leukocyte-reduced by filtration were able to induce tolerance to control platelet transfusions from the 2° and 3° donors to whom they had never previously been exposed (p<0.001, compared to all other platelet products given). In other experiments, if plasma free red cells that had been leuko-reduced by centrifugation and filtration or plasma that had been leuko-reduced by centrifugation and filtration were added to the primary donor dog's platelets that had been leuko-reduced by centrifugation and filtration, these platelet products were still non-immunogenic. RBCs for these experiments were prepared by filtering packed red cells (platelet-rich plasma removed) through two BPF-4 filters, centrifuging the filtered RBCs at 1300 xg for 15 minutes and removing the bottom 1-2 cc of RBCs. The RBCs were then washed with saline twice. To prepare the plasma, whole blood was spun at 1300 xg for 10 minutes. The supernatant plasma was removed and filtered through a PLF-1 filter.

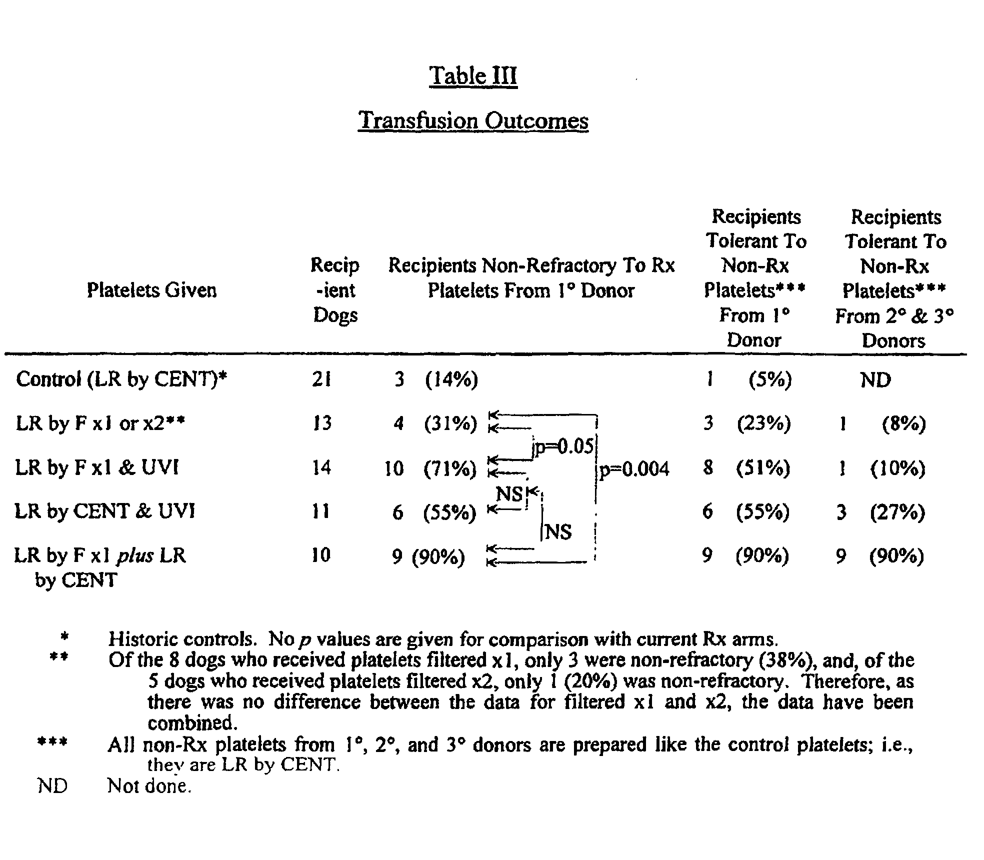

### Example 4

### Induction of Immunologic Tolerance

This Example illustrates the use of platelet compositions described in Examples 1 and 2 to induce immunologic tolerance. Of the subgroup of 29 animals in Table III (lines 2-5) which did not become refractory to treated platelets from their primary donors, only 3 (10%) subsequently became refractory to their primary donor's platelets which were leukocyte-reduced by centrifugation. This rate of refractoriness is much lower than the 86% platelet refractory rate seen when this product was given as the initial platelet treatment program (Table III, line 1), suggesting tolerance induction by prior treatment with filtered, filtered-UVI, centrifuged-UYI, and filtered-centrifuged products. In subsequent experiments, it has been determined that animals which have received doubly modified products are tolerant to unmodified platelet transfusions from their primary donor. Such doubly modified platelet and red blood cell products may also be used to induce tolerance to other transfused and transplanted tissues, particularly the centrifuged and filtered leukocyte-reduced platelet and red blood cell products.

## Claims

1. A method of preparing a non-immunogenic and toleragenic platelet composition, comprising:
(a) centrifuging a platelet-rich blood product one or more times and recovering the supernatant; and
(b) filtering the supernatant and recovering the filtrate to produce a non-immunogenic and toleragenic platelet composition.

2. A method according to claim 1, wherein the step of centrifuging comprises a first soft spin and a second soft spin, wherein said soft spin comprises centrifugation at approximately 180 to 2000 G.

3. A method according to claim 2, wherein the step of centrifuging further comprises a hard spin to produce a platelet concentrate, and wherein said hard spin comprises centrifugation at approximately 500 to 6000 G.

4. A method according to claim 1 wherein the step of centrifuging comprises at least one soft spin and a hard spin, and wherein said soft and hard spins comprise centrifugation at approximately 180 to 2000 G and 500 to 6000 G, respectively, and they are performed prior to the step of filtering.

5. A method according to claim 2 wherein the step of filtering is performed after the first soft spin and before the second soft spin.

6. A method of preparing a non-immunogenic and toleragenic platelet composition, comprising:
performing a first soft spin wherein said soft spin comprises centrifugation at approximately 180 to 2000 G on a platelet-rich blood product and recovering a supernatant platelet composition, and thereafter, in any order, performing the following steps:
(1) performing a second soft spin wherein said soft spin comprises centrifugation at approximately 180 to 2000 G on the platelet composition and recovering a supernatant platelet composition;
(2) performing a hard spin wherein said hard spin comprises centrifugation at approximately 500 to 6000 G on the platelet composition and recovering a supernatant platelet composition; and
(3) filtering the platelet composition and recovering a filtrate platelet composition; thereby producing a non-immunogenic and toleragenic platelet composition.

7. A method of preparing a non-immunogenic and toleragenic platelet composition comprising:
(a) filtering a platelet-rich blood product and recovering the filtrate; and
(b) Subsequently centrifuging the filtrate and recovering the supernatant, to produce a non-immunogenic and toleragenic platelet composition.

8. A method according to any one of claims 1, 6, or 7 wherein the platelet-rich blood product is selected from the group consisting of whole blood, platelet-rich plasma, platelet concentrates , apheresis platelets and buffy-coat platelet preparations.

9. A non-immunogenic and toleragenic platelet composition, said composition being substantially free of white blood cells for use in a method of inducing immunologic tolerance in a patient to transfused or transplanted tissue comprising administering to the patient said composition in an amount sufficient to induce immunologic tolerance to subsequently transfused or transplanted tissue.

10. A method according to claim 9 wherein the transfused or transplanted tissue is selected from the group consisting of bone marrow, skin, pancreas, bone, liver, heart, lung, kidney and cornea.

11. A method according to claim 9 wherein the transfused or transplanted tissue is blood.

12. A method of preparing a non-immunogenic and toleragenic red blood cell composition, comprising:
(a) centrifuging a red blood cell-rich blood product and recovering the lower layer; and
(b) subsequently filtering the lower layer and recovering the filtrate to produce a non-immunogenic and toleragenic red blood cell composition.

13. A method according to claim 12, wherein the step of centrifuging comprises a hard spin wherein said hard spin comprises centrifugation at approximately 500 to 6000 G.

14. A method according to claim 3 wherein the hard spin is performed prior to the second soft spin.

15. A method according to claim 3 wherein the hard spin is performed after the second soft spin.

16. A method according to claim 3 wherein the hard spin is performed prior to the first soft spin.

17. A platelet composition obtainable from a platelet-rich blood product according to steps comprising:
(a) centrifuging the platelet-rich blood product one or more times and recovering the supernatant; and
(b) filtering the supernatant and recovering the filtrate to produce a non-immunogenic and toleragenic platelet composition.

18. The platelet composition according to claim 17, wherein the step of centrifuging comprises a first soft spin and a second soft spin, wherein said soft spin comprises centrifugation at approximately 180 to 2000 G.

19. The platelet composition according to claim 18, wherein the step of centrifuging further comprises a hard spin to produce a platelet concentrate, and wherein said hard spin comprises centrifugation at approximately 500 to 6000 G.

20. The platelet composition according to claim 17 wherein the step of centrifuging comprises at least one soft spin and a hard spin, and wherein said soft and hard spins comprise centrifugation at approximately 180 to 2000 G and 500 to 6000 G, respectively, and they are performed prior to the step of filtering.

21. A platelet composition obtainable from a platelet-rich blood product according to steps comprising:
(a) filtering the platelet-rich blood product and recovering the filtrate; and
(b) centrifuging the filtrate and recovering the supernatant to produce a non-immunogenic and toleragenic platelet composition.

22. The platelet composition according to claim 21, wherein the step of centrifuging comprises a first soft spin and a second soft spin, wherein said soft spin comprises centrifugation at approximately 180 to 2000 G.

23. The platelet composition according to claim 22, wherein the step of centrifuging further comprises a hard spin to produce a platelet concentrate, and wherein said hard spin comprises centrifugation at approximately 500 to 6000 G.

24. The platelet composition according to claim 21 wherein the step of centrifuging comprises at least one soft spin and a hard spin, and wherein said soft and hard spins comprise centrifugation at approximately 180 to 2000 G and 500 to 6000 G, respectively, and they are performed prior to the step of filtering.

## Patentansprüche

1. Verfahren zur Herstellung einer nicht-immunogenen und toleragenen Blutplättchen-Zusammensetzung, umfassend:
(a) Zentrifugieren eines Blutplättchen-reichen Blutprodukts ein oder mehrere Male und Zurückerhalten des Überstands; und
(b) Filtrieren des Überstands und Zurückerhalten des Filtrats, um eine nicht-immunogene und toleragene Blutpättchen-Zusammensetzung herzustellen.

2. Verfahren nach Anspruch 1, wobei der Schritt des Zentrifugierens ein erstes sanftes Zentrifugieren und ein zweites sanftes Zentrifugieren umfaßt, wobei das sanfte Zentrifugieren eine Zentrifugation bei ungefähr 180 bis 2000 g umfaßt.

3. Verfahren nach Anspruch 2, wobei der Schritt des Zentrifugieren weiterhin ein starkes Zentrifugieren umfaßt, um so ein Plättchenkonzentrat herzustellen, und wobei das starke Zentrifugieren ein Zentrifugieren bei ungefähr 500 bis 6000 g umfaßt.

4. Verfahren nach Anspruch 1, wobei der Schritt des Zentrifugierens mindestens ein sanftes Zentrifugieren und ein starkes Zentrifugieren umfaßt, und wobei die sanften und starken Zentrifugationen eine Zentrifugation bei jeweils etwa 180 bis 2000 g und 500 bis 6000 g umfassen und sie vor dem Schritt der Filtrierung durchgeführt werden.

5. Verfahren nach Anspruch 2, wobei der Schritt des Filtrierens nach dem ersten sanften Zentrifugieren und vor dem zweiten sanften Zentrifugieren durchgerührt wird.

6. Verfahren zur Herstellung einer nicht-immunogenen und toleragenen Blutplättchen-Zusammensetzung umfassend:
Durchführen eines ersten sanften Zentrifugierens, wobei das sanfte Zentrifugieren ein Zentrifugieren bei ungefähr 180 bis 2000 g mit einem Blutplättchen-reichen Produkt umfaßt und Zurückerhalten einer Überstand-Blutplättchen-Zusammensetzung, und danach Durchführen der folgenden Schritte in beliebiger Reihenfolge:
(1) Durchführen eines zweiten sanften Zentrifugierens, wobei das sanfte Zentrifugieren ein Zentrifugieren bei ungefähr 180 bis 2000 g mit der Blutplättchen-Zusammensetzung umfaßt und Zurückerhalten einer Überstand-Blutplättchen-Zusammensetzung;
(2) Durchführen eines starken Zentrifugierens, wobei das starke Zentrifugieren ein Zentrifugieren bei ungefähr 500 bis 6000 g mit der Blutplättchen-Zusammensetzung umfaßt und Zurückerhalten einer Überstand-Blutplättchen-Zusammensetzung; und
(3) Filtrieren der Blutplättchen-Zusammensetzung und Zurückerhalten einer Filtrat-Blutplättchen-Zusammensetzung; wodurch eine nicht-immunogene und toleragene Blutplättchen-Zusammensetzung hergestellt wird.

7. Verfahren zur Herstellung einer nicht-immunogenen und toleragenen Blutplättchen-Zusammensetzung, umfassend:
(a) Filtrieren eines Blutplättchen-reichen Produkts und Zurückerhalten des Filtrats; und
(b) anschließendes Zentrifugieren des Filtrats und Zurückerhalten des Überstands, um eine nicht-immunogene und toleragene Blutplättchen-Zusammensetzung herzustellen.

8. Verfahren nach einem der Ansprüche 1, 6 oder 7, worin das Blutplättchen-reiche Produkt ausgewählt ist aus der Gruppe bestehend aus Gesamtblut, Blutplättchen-reichem Plasma, Blutplättchen-Konzentraten, Apherese-Blutplättchen und Leukozytenfilmbeschichtete Blutplättchen-Präparate.

9. Nicht-immunogene und toleragene Blutplättchen-Zusammensetzung, wobei die Zusammensetzung im wesentlichen frei von weißen Blutzellen ist, zur Verwendung in einem Verfahren zur Induktion von immunologischer Toleranz in einem Patienten gegenüber Transfusions- oder Transplantationsgewebe, umfassend Verabreichen der Zusammensetzung an einen Patienten in einer Menge, die ausreichend ist, um immunogene Toleranz gegenüber anschließend transfundiertem oder transplantiertem Gewebe zu induzieren.

10. Zusammensetzung nach Anspruch 9, wobei das transfundierte oder transplantierte Gewebe ausgewählt ist aus der Gruppe bestehend aus Knochenmark, Haut, Pankreas, Knochen, Leber, Herz, Lunge, Niere und Hornhaut.

11. Zusammensetzung nach Anspruch 9, wobei das transfundierte oder transplantierte Gewebe Blut ist.

12. Verfahren zur Herstellung einer nicht-immunogenen und toleragenen rote Blutzellenzusammensetzung, umfassend:
(a) Zentrifugieren eines roten Blutzellen-reichen Blutprodukts und Zurückerhalten der unteren Lage; und
(b) anschließendes Filtrieren der unteren Lage und Zurückerhalten des Filtrats, um eine nicht-immunogene und toleragene rote Blutzellen-Zusammensetzung herzustellen.

13. Verfahren nach Anspruch 12, wobei der Schritt von Zentrifugieren ein starkes Zentrifugieren umfaßt, wobei das starke Zentrifugieren ein Zentrifugieren bei ungefähr 500 bis 6000 g umfaßt.

14. Verfahren nach Anspruch 3, wobei das starke Zentrifugieren vor dem zweiten sanften Zentrifugieren durchgeführt wird.

15. Verfahren nach Anspruch 3, wobei das starke Zentrifugieren nach dem zweiten sanften Zentrifugieren durchgeführt wird.

16. Verfahren nach Anspruch 3, wobei das starke Zentrifugieren vor dem ersten sanften Zentrifugieren durchgeführt wird.

17. Blutplättchen-Zusammensetzung, erhältlich aus einem Blutplättchen-reichen Blutprodukt gemäß den Schritten, die umfassen:
(a) Zentrifugieren des Blutplättchen-reichen Blutprodukts ein oder mehrere Male und Zurückerhalten des Überstands;
(b) Filtrieren des Überstands und Zurückerhalten des Überstands, um eine nicht-immunogene und toleragene Blutplättchen-Zusammensetzung herzustellen.

18. Zusammensetzung gemäß Anspruch 17, worin der Schritt zum Zentrifugieren ein erstes sanftes und ein zweites sanftes Zentrifugieren umfaßt, wobei das sanfte Zentrifugieren eine Zentrifugation bei ungefähr 180 bis 2000 g umfaßt.

19. Blutplättchen-Zusammensetzung nach Anspruch 18, worin der Schritt des Zentrifugierens weiterhin ein starkes Zentrifugieren umfaßt, um ein Blutplättchen-Konzentrat zu ergeben, und worin das starke Zentrifugieren eine Zentrifugation bei ungefähr 500 bis 6000 g umfaßt.

20. Blutplättchen-Zusammensetzung nach Anspruch 17, wobei der Schritt des Zentrifugierens mindestens ein sanftes Zentrifugieren und ein starkes Zentrifugieren umfaßt und wobei die sanften und starken Zentrifugationen jeweils eine Zentrifugation bei ungefähr 180 bis 2000 g und 500 bis 6000 g umfassen, und sie vor dem Filtrationsschritt durchgeführt werden.

21. Blutplättchen-Zusammensetzung, erhältlich aus einem Blutplättchen-reichen Blutprodukt gemäß den Schritten, die umfassen:
(a) Filtrieren des Blutplättchen-reichen Blutprodukts und Zurückerhalten des Filtrats; und
(b) Zentrifugieren des Filtrats und Zurückbehalten des Überstands, um eine nicht-immunogene und toleragene Blutplättchen-Zusammensetzung herzustellen.

22. Blutplättchen-Zusammensetzung gemäß Anspruch 21, wobei der Schritt des Zentrifugieren ein erstes sanftes und ein zweites sanftes Zentrifugieren umfaßt, wobei das sanfte Zentrifugieren eine Zentrifügation bei ungefähr 180 bis 2000 g umfaßt.

23. Blutplättchen-Zusammensetzung nach Anspruch 22, wobei der Schritt des Zentrifügierens weiterhin ein starkes Zentrifugieren umfaßt, um ein Blutplättchen-Konzentrat zu ergeben und wobei das starke Zentrifugieren eine Zentrifugation bei ungefähr 500 bis 6000 g umfaßt.

24. Blutplättchen-Zusammensetzung nach Anspruch 21, wobei der Schritt des Zentrifugierens mindestens ein sanftes und ein starkes Zentrifugieren umfaßt und wobei das sanfte und harte Zentrifugieren jeweils eine Zentrifugation bei 180 bis 2000 g und 500 bis 6000 g umfaßt, und diese vor dem Schritt des Filtrierens durchgeführt werden.

## Revendications

1. Procédé pour la préparation d'une composition de plaquettes non immunogène et engendrant une tolérance, comprenant les étapes consistant :
(a) à centrifuger un produit sanguin riche en plaquettes une ou plusieurs fois et à recueillir le surnageant ; et
(b) à filtrer le surnageant et recueillir le filtrat pour produire une composition de plaquettes non immunogène et engendrer une tolérance.

2. Procédé suivant la revendication 1, dans lequel l'étape de centrifugation comprend un premier mouvement rotatif doux et un second mouvement rotatif doux, ledit mouvement rotatif doux comprenant une centrifugation à approximativement 180 à 2000 x g.

3. Procédé suivant la revendication 2, dans lequel l'étape de centrifugation comprend en outre un mouvement rotatif intense pour produire un concentré de plaquettes, ledit mouvement rotatif intense comprenant une centrifugation à approximativement 500 à 6000 x g.

4. Procédé suivant la revendication 1, dans lequel l'étape de centrifugation comprend au moins un mouvement rotatif doux et un mouvement rotatif intense, ledit mouvement rotatif doux et ledit mouvement rotatif intense comprenant des centrifugations respectivement à approximativement 180 à 2000 x g et 500 à 6000 x g, dont la mise en oeuvre est effectuée avant l'étape de filtration.

5. Procédé suivant la revendication 2, dans lequel l'étape de filtration est mise en oeuvre après le premier mouvement rotatif doux et avant le second mouvement rotatif doux.

6. Procédé pour la préparation d'une composition de plaquettes non immunogène et engendrant une tolérance, comprenant les étapes consistant :
à effectuer un premier mouvement rotatif doux, ledit mouvement rotatif doux comprenant une centrifugation à approximativement 180 à 2000 x g sur un produit sanguin riche en plaquettes et à recueillir une composition de plaquettes surnageante, et ensuite, dans n'importe quel ordre, à mettre en oeuvre les étapes consistant :
(1) à effectuer un second mouvement rotatif doux, ledit second mouvement rotatif doux comprenant une centrifugation à approximativement 180 à 2000 x g sur la composition de plaquettes, et à recueillir une composition de plaquettes surnageante ;
(2) à effectuer un mouvement rotatif intense, ledit mouvement rotatif intense comprenant une centrifugation à approximativement 500 à 6000 x g sur la composition de plaquettes, et à recueillir une composition de plaquettes surnageante ; et
(3) à filtrer la composition de plaquettes et à recueillir une composition de plaquettes comme filtrat ; en produisant ainsi une composition de plaquettes non immunogène et engendrant une tolérance.

7. Procédé pour la préparation d'une composition, de plaquettes non immunogène et engendrant une tolérance, comprenant les étapes consistant :
(a) à filtrer un produit sanguin riche en plaquettes et à recueillir le filtrat ; et
(b) ensuite à centrifuger le filtrat et recueillir le surnageant, pour produire une composition de plaquettes non immunogène et engendrant une tolérance.

8. Procédé suivant l'une quelconque des revendications 1, 6 et 7, dans lequel le produit sanguin riche en plaquettes est choisi dans le groupe consistant en le sang entier, le plasma riche en plaquettes, des concentrés de plaquettes, des plaquettes d'aphérèse et des préparations de plaquettes de couches leucocytaires.

9. Composition de plaquettes non immunogène et engendrant une tolérance, ladite composition étant pratiquement dépourvue de globules blancs, destinée à être utilisée dans une méthode pour induire une tolérance immunologique chez un patient vis-à-vis d'un tissu transfusé ou transplanté, comprenant l'administration au patient de ladite composition en une quantité suffisante pour induire une tolérance immunologique vis-à-vis du tissu ultérieurement transfusé ou transplanté.

10. Composition suivant la revendication 9, dans laquelle le tissu transfusé ou transplanté est choisi dans le groupe consistant en la moelle osseuse, la peau, le pancréas, le tissu osseux, le foie, le coeur, le poumon, le rein et la cornée.

11. Composition suivant la revendication 9, dans laquelle le tissu transfusé ou transplanté est le sang.

12. Procédé pour la préparation d'une composition de globules rouges non immunogène et engendrant une tolérance, comprenant les étapes consistant :
(a) à centrifuger un produit sanguin riche en globules rouges et à recueillir la couche inférieure ; et
(b) ensuite à filtrer la couche inférieure et recueillir le filtrat pour produire une composition de globules rouges non immunogène et engendrant une tolérance.

13. Procédé suivant la revendication 12, dans lequel l'étape de centrifugation comprend un mouvement rotatif intense, ledit mouvement rotatif intense comprenant une centrifugation à approximativement 500 à 6000 x g.

14. Procédé suivant la revendication 3, dans lequel le mouvement rotatif intense est effectué avant le second mouvement rotatif doux.

15. Procédé suivant la revendication 3, dans lequel le mouvement rotatif intense est effectué après le second mouvement rotatif doux.

16. Procédé suivant la revendication 3, dans lequel le mouvement rotatif intense est effectué avant le premier mouvement rotatif doux.

17. Composition de plaquettes pouvant être obtenue à partir d'un produit sanguin riche en plaquettes par des étapes consistant :
(a) à centrifuger le produit sanguin riche en plaquettes une ou plusieurs fois et à recueillir le surnageant ; et
(b) à filtrer le surnageant et recueillir le filtrat pour produire une composition de plaquettes non immunogène et engendrant une tolérance.

18. Composition de plaquettes suivant la revendication 17, dans laquelle l'étape de centrifugation comprend un premier mouvement rotatif doux et un second mouvement rotatif doux, ledit mouvement rotatif doux comprenant une centrifugation à approximativement 180 à 2000 x g.

19. Composition de plaquettes suivant la revendication 18, dans laquelle l'étape de centrifugation comprend en outre un mouvement rotatif intense pour produire un concentré de plaquettes, ledit mouvement rotatif intense comprenant une centrifugation à approximativement 500 à 6000 x g.

20. Composition de plaquettes suivant la revendication 17, dans laquelle l'étape de centrifugation comprend au moins un mouvement rotatif doux et un mouvement rotatif intense, ledit mouvement rotatif doux et ledit mouvement rotatif intense comprenant respectivement des centrifugations à approximativement 180 à 2000 x g et 500 à 6000 x g et étant effectuées avant l'étape de filtration.

21. Composition de plaquettes pouvant être obtenue à partir d'un produit sanguin riche en plaquettes par les étapes consistant :
(a) à filtrer le produit sanguin riche en plaquettes et à recueillir le filtrat ; et
(b) à centrifuger le filtrat et recueillir le surnageant pour produire une composition de plaquettes non immunogène et engendrant une tolérance.

22. Composition de plaquettes suivant la revendication 21, dans laquelle l'étape de centrifugation comprend un premier mouvement rotatif doux et un second mouvement rotatif doux, ledit mouvement rotatif doux comprenant une centrifugation à approximativement 180 à 2000 x g.

23. Composition de plaquettes suivant la revendication 22, dans laquelle l'étape de centrifugation comprend en outre un mouvement rotatif intense pour produire un concentré de plaquettes, ledit mouvement rotatif intense comprenant une centrifugation à approximativement 500 à 6000 x g.

24. Composition de plaquettes suivant la revendication 21, dans laquelle l'étape de centrifugation comprend au moins un mouvement rotatif doux et un mouvement rotatif intense, ledit mouvement rotatif doux et ledit mouvement rotatif intense comprenant respectivement des centrifugations à approximativement 180 à 2000 x g et 500 à 6000 x g et étant effectuées avant l'étape de filtration.
